# EUROPÄISCHE PATENTSCHRIFT

(11) **EP 1 929 311 B1**
(45) Veröffentlichungstag und Bekanntmachung des Hinweises auf die Patenterteilung: **31.03.2010**
(21) Anmeldenummer: 06806812.1
(22) Anmeldetag: 25.09.2006
(51) Int. Cl.: G01N 33/76, G01N 33/564, C07K 14/72

(54) **VERFAHREN ZUM NACHWEIS VON AUTOIMMUNANTIKÖRPERN GEGEN DEN TSH-REZEPTOR UND NEUE TSH- REZEPTORCHIMÄREN**
METHOD FOR DETECTING AUTOIMMUNE ANTIBODIES AGAINST TSH RECEPTORS AND NOVEL TSH-RECEPTOR CHIMAERAS
PROCEDE D'IDENTIFICATION D'AUTOANTICORPS CONTRE LE RECEPTEUR DE LA TSH ET NOUVEAUX CHIMERES DU RECEPTEUR DE LA TSH

(30) Priorität: 26.09.2005 DE 102005046022
(43) Veröffentlichungstag der Anmeldung: 11.06.2008
(73) Patentinhaber: Loos, Ulrich, 89081 Ulm (DE)
(72) Erfinder: Loos, Ulrich, 89081 Ulm (DE)
(74) Vertreter: Cohausz & Florack
(86) Internationale Anmeldenummer: PCT/EP2006/066719
(87) Internationale Veröffentlichungsnummer: WO 2007/036511

(56) Entgegenhaltungen:
- EP-A- 1 078 986
- WO-A-00/00590
- WO-A2-01/63296
- DE-C1- 19 645 729
- TAHARA K ET AL: "EPITOPES FOR THYROID STIMULATING AND BLOCKING AUTOANTIBODIES ON THEEXTRACELLULAR DOMAIN OF THE HUMAN THYROTROPIN RECEPTOR" THYROID, MARY ANN LIEBERT, NEW YORK, NY, US, Bd. 7, Nr. 6, Dezember 1997 (1997-12), Seiten 867-877, XP000993366 ISSN: 1050-7256

### SEQUENCE LISTING

<110> Loos, Ulrich
<120> Innovativer TSH-R-Ab Kit
<130> 051314WO
<150> 102005046022.4
   <151> 2005-09-26
<160> 10
<170> PatentIn version 3.3
<210> 1
   <211> 2319
   <212> DNA
   <213> Homo sapiens
<400> 1
   atgctgctgc tgctgctgct gctgggcctg aggctacagc tctccctggg catcatccca      60
   gttgaggagg agaacccgga cttctggaac cgcgaggcag ccgaggccct gggtgccgcc     120
   aagaagctgc agcctgcaca gacagccgcc aagaacctca tcatcttcct gggcgatggg     180
   atgggggtgt ctacggtgac agctgccagg atcctaaaag ggcagaagaa ggacaaactg     240
   gggcctgaga tacccctggc catggaccgc ttcccatatg tggctctgtc caagacatac     300
   aatgtagaca aacatgtgcc agacagtgga gccacagcca cggcctacct gtgcggggtc     360
   aagggcaact tccagaccat tggcttgagt gcagccgccc gctttaacca gtgcaacacg     420
   acacgcggca acgaggtcat ctccgtgatg aatcgggcca agaaagcagg gaagtcagtg     480
   ggagtggtaa ccaccacacg agtgcagcac gcctcgccag ccggcaccta cgcccacacg     540
   gtgaaccgca actggtactc ggacgccgac gtgcctgcct cggcccgcca ggaggggtgc     600
   caggacatcg ctacgcagct catctccaac atggacattg acgtgatcct aggtggaggc     660
   cgaaagtaca tgtttcgcat gggaacccca gaccctgagt acccagatga ctacagccaa     720
   ggtgggacca ggctggacgg gaagaatctg gtgcaggaat ggctggcgaa gcgccagggt     780
   gcccggtatg tgtggaaccg cactgagctc atgcaggctt ccctggaccc gtctgtgacc     840
   catctcatgg gtctctttga gcctggagac atgaaatacg agatccaccg agactccaca     900
   ctggacccct ccctgatgga gatgacagag gctgccctgc gcctgctgag caggaacccc     960
   cgcggcttct tcctcttcgt ggagggtggt cgcatcgacc atggtcatca tgaaagcagg    1020
   gcttaccggg cactgactga gacgatcatg ttcgacgacg ccattgagag ggcgggccag    1080
   ctcaccagcg aggaggacac gctgagcctc gtcactgccg accactccca cgtcttctcc    1140
   ttcggaggct accccctgcg agggagctcc atcttcgggc tggcccctgg caaggcccgg    1200
   gacaggaagg cctacacggt cctcctatac ggaaacggtc caggctatgt gctcaaggac    1260
   ggcgcccggc cggatgttac cgagagcgag agcgggagcc ccgagtatcg gcagcagtca    1320
   gcagtgcccc tggacgaaga gacccacgca ggcgaggacg tggcggtgtt cgcgcgcggc    1380
   ccgcaggcgc acctggttca cggcgtgcag gagcagacct tcatagcgca cgtcatggcc    1440
   ttcgccgcct gcctggagcc ctacaccgcc tgcgacctgg cgccccccgc cggcaccacc   1500
   gacgccgcgc acccgggtta ctctagagtc ggggcggccg gccgcttcga gcagacagga   1560
   atggggtgtt cgtctccacc ctgcgagtgc catcaggagg aggacttcag agtcacctgc   1620
   aaggatattc aacgcatccc cagcttaccg cccagtacgc agactctgaa gcttattgag   1680
   actcacctga gaactattcc aagtcatgca ttttctaatc tgcccaatat ttccagaatc   1740
   tacgtatcta tagatgtgac tctgcagcag ctggaatcac actccttcta caatttgagt   1800
   aaagtgactc acatagaaat tcggaatacc aggaacttaa cttacataga ccctgatgcc   1860
   ctcaaagagc tccccctcct aaagttcctt ggcattttca acactggact taaaatgttc   1920
   cctgacctga ccaaagttta ttccactgat atattcttta tacttgaaat tacagacaac   1980
   ccttacatga cgtcaatccc tgtgaatgct tttcagggac tatgcaatga aaccttgaca   2040
   ctgaagctgt acaacaatgg ctttacttca gtccaaggat atgctttcaa tgggacaaag   2100
   ctggatgctg tttacctaaa caagaataaa tacctgacag ttattgacaa agatgcattt   2160
   ggaggagtat acagtggacc aagcttgctg gacgtgtctc aaaccagtgt cactgccctt   2220
   ccatccaaag gcctggagca cctgaaggaa ctgatagcaa gaaacacctg gactcttaag   2280
   aaaacactgc cctccaaaca tcaccatcac catcactaa                          2319
<210> 2
   <211> 772
   <212> PRT
   <213> Homo sapiens
<400> 2
   Met Leu Leu Leu Leu Leu Leu Leu Gly Leu Arg Leu Gln Leu Ser Leu
   1               5                   10                  15
   Gly Ile Ile Pro Val Glu Glu Glu Asn Pro Asp Phe Trp Asn Arg Glu
               20                  25                  30
   Ala Ala Glu Ala Leu Gly Ala Ala Lys Lys Leu Gln Pro Ala Gln Thr
           35                  40                  45
   Ala Ala Lys Asn Leu Ile Ile Phe Leu Gly Asp Gly Met Gly Val Ser
       50                  55                  60
   Thr Val Thr Ala Ala Arg Ile Leu Lys Gly Gln Lys Lys Asp Lys Leu
   65                  70                  75                  80
   Gly Pro Glu Ile Pro Leu Ala Met Asp Arg Phe Pro Tyr Val Ala Leu
                   85                  90                  95
   Ser Lys Thr Tyr Asn Val Asp Lys His Val Pro Asp Ser Gly Ala Thr
               100                 105                 110
   Ala Thr Ala Tyr Leu Cys Gly Val Lys Gly Asn Phe Gln Thr Ile Gly
           115                 120                 125
   Leu Ser Ala Ala Ala Arg Phe Asn Gln Cys Asn Thr Thr Arg Gly Asn
       130                 135                 140
   Glu Val Ile Ser Val Met Asn Arg Ala Lys Lys Ala Gly Lys Ser Val
   145                 150                 155                 160
   Gly Val Val Thr Thr Thr Arg Val Gln His Ala Ser Pro Ala Gly Thr
                   165                 170                 175
   Tyr Ala His Thr Val Asn Arg Asn Trp Tyr Ser Asp Ala Asp Val Pro
               180                 185                 190
   Ala Ser Ala Arg Gln Glu Gly Cys Gln Asp Ile Ala Thr Gln Leu Ile
           195                 200                 205
   Ser Asn Met Asp Ile Asp Val Ile Leu Gly Gly Gly Arg Lys Tyr Met
       210                 215                 220
   Phe Arg Met Gly Thr Pro Asp Pro Glu Tyr Pro Asp Asp Tyr Ser Gln
   225                 230                 235                 240
   Gly Gly Thr Arg Leu Asp Gly Lys Asn Leu Val Gln Glu Trp Leu Ala
                   245                 250                 255
   Lys Arg Gln Gly Ala Arg Tyr Val Trp Asn Arg Thr Glu Leu Met Gln
               260                 265                 270
   Ala Ser Leu Asp Pro Ser Val Thr His Leu Met Gly Leu Phe Glu Pro
           275                 280                 285
   Gly Asp Met Lys Tyr Glu Ile His Arg Asp Ser Thr Leu Asp Pro Ser
       290                 295                 300
   Leu Met Glu Met Thr Glu Ala Ala Leu Arg Leu Leu Ser Arg Asn Pro
   305                 310                 315                 320
   Arg Gly Phe Phe Leu Phe Val Glu Gly Gly Arg Ile Asp His Gly His
                   325                 330                 335
   His Glu Ser Arg Ala Tyr Arg Ala Leu Thr Glu Thr Ile Met Phe Asp
               340                 345                 350
   Asp Ala Ile Glu Arg Ala Gly Gln Leu Thr Ser Glu Glu Asp Thr Leu
           355                 360                 365
   Ser Leu Val Thr Ala Asp His Ser His Val Phe Ser Phe Gly Gly Tyr
       370                 375                 380
   Pro Leu Arg Gly Ser Ser Ile Phe Gly Leu Ala Pro Gly Lys Ala Arg
   385                 390                 395                 400
   Asp Arg Lys Ala Tyr Thr Val Leu Leu Tyr Gly Asn Gly Pro Gly Tyr
   405 410 415
   Val Leu Lys Asp Gly Ala Arg Pro Asp Val Thr Glu Ser Glu Ser Gly
   420 425 430
   Ser Pro Glu Tyr Arg Gln Gln Ser Ala Val Pro Leu Asp Glu Glu Thr
   435 440 445
   His Ala Gly Glu Asp Val Ala Val Phe Ala Arg Gly Pro Gln Ala His
   450 455 460
   Leu Val His Gly Val Gln Glu Gln Thr Phe Ile Ala His Val Met Ala
   465 470 475 480
   Phe Ala Ala Cys Leu Glu Pro Tyr Thr Ala Cys Asp Leu Ala Pro Pro
   485 490 495
   Ala Gly Thr Thr Asp Ala Ala His Pro Gly Tyr Ser Arg Val Gly Ala
   500 505 510
   Ala Gly Arg Phe Glu Gln Thr Gly Met Gly Cys Ser Ser Pro Pro Cys
   515 520 525
   Glu Cys His Gln Glu Glu Asp Phe Arg Val Thr Cys Lys Asp Ile Gln
   530 535 540
   Arg Ile Pro Ser Leu Pro Pro Ser Thr Gln Thr Leu Lys Leu Ile Glu
   545 550 555 560
   Thr His Leu Arg Thr Ile Pro Ser His Ala Phe Ser Asn Leu Pro Asn
   565 570 575
   Ile Ser Arg Ile Tyr Val Ser Ile Asp Val Thr Leu Gln Gln Leu Glu
   580 585 590
   Ser His Ser Phe Tyr Asn Leu Ser Lys Val Thr His Ile Glu Ile Arg
   595 600 605
   Asn Thr Arg Asn Leu Thr Tyr Ile Asp Pro Asp Ala Leu Lys Glu Leu
   610 615 620
   Pro Leu Leu Lys Phe Leu Gly Ile Phe Asn Thr Gly Leu Lys Met Phe
   625 630 635 640
   Pro Asp Leu Thr Lys Val Tyr Ser Thr Asp Ile Phe Phe Ile Leu Glu
   645 650 655
   Ile Thr Asp Asn Pro Tyr Met Thr Ser Ile Pro Val Asn Ala Phe Gln
   660 665 670
   Gly Leu Cys Asn Glu Thr Leu Thr Leu Lys Leu Tyr Asn Asn Gly Phe
   675 680 685
   Thr Ser Val Gln Gly Tyr Ala Phe Asn Gly Thr Lys Leu Asp Ala Val
   690 695 700
   Tyr Leu Asn Lys Asn Lys Tyr Leu Thr Val Ile Asp Lys Asp Ala Phe
   705 710 715 720
   Gly Gly Val Tyr Ser Gly Pro Ser Leu Leu Asp Val Ser Gln Thr Ser
   725 730 735
   Val Thr Ala Leu Pro Ser Lys Gly Leu Glu His Leu Lys Glu Leu Ile
   740 745 750
   Ala Arg Asn Thr Trp Thr Leu Lys Lys Thr Leu Pro Ser Lys His His
   755 760 765
   His His His His
   770
<210> 3
   <211> 849
   <212> DNA
   <213> Homo sapiens
<400> 3
   atgaggccgg cggacttgct gcagctggtg ctgctgctcg acctgcccag ggacctgggc 60
   ggaatggggt gttcgtctcc accctgcgag tgccatcagg aggaggactt cagagtcacc 120
   tgcaaggata ttcaacgcat ccccagctta ccgcccagta cgcagactct gaagcttatt 180
   gagactcacc tgagaactat tccaagtcat gcattttcta atctgcccaa tatttccaga 240
   atctacgtat ctatagatgt gactctgcag cagctggaat cacactcctt ctacaatttg 300
   agtaaagtga ctcacataga aattcggaat accaggaact taacttacat agaccctgat 360
   gccctcaaag agctccccct cctaaagttc cttggcattt tcaacactgg acttaaaatg 420
   ttccctgacc tgaccaaagt ttattccact gatatattct ttatacttga aattacagac 480
   aacccttaca tgacgtcaat ccctgtgaat gcttttcagg gactatgcaa tgaaaccttg 540
   acactgaagc tgtacaacaa tggctttact tcagtccaag gatatgcttt caatgggaca 600
   aagctggatg ctgtttacct aaacaagaat aaatacctga cagttattga caaagatgca 660
   tttggaggag tatacagtgg accaagcttg ctggacgtgt ctcaaaccag tgtcactgcc 720
   cttccatcca aaggcctgga gcacctgaag gaactgatag caagaaacac ctggactctt 780
   aaggaaaaat cccatctaac cccaaagaag caaggccaaa tctcagaaga gtatatgcaa 840
   acggtttaa 849
<210> 4
   <211> 282
   <212> PRT
   <213> Homo sapiens
<400> 4
   Met Arg Pro Ala Asp Leu Leu Gln Leu Val Leu Leu Leu Asp Leu Pro
   1               5                   10                  15
   Arg Asp Leu Gly Gly Met Gly Cys Ser Ser Pro Pro Cys Glu Cys His
               20                  25                  30
   Gln Glu Glu Asp Phe Arg Val Thr Cys Lys Asp Ile Gln Arg Ile Pro
           35                  40                  45
   Ser Leu Pro Pro Ser Thr Gln Thr Leu Lys Leu Ile Glu Thr His Leu
       50                  55                  60
   Arg Thr Ile Pro Ser His Ala Phe Ser Asn Leu Pro Asn Ile Ser Arg
   65                  70                  75                  80
   Ile Tyr Val Ser Ile Asp Val Thr Leu Gln Gln Leu Glu Ser His Ser
                   85                  90                  95
   Phe Tyr Asn Leu Ser Lys Val Thr His Ile Glu Ile Arg Asn Thr Arg
               100                 105                 110
   Asn Leu Thr Tyr Ile Asp Pro Asp Ala Leu Lys Glu Leu Pro Leu Leu
           115                 120                 125
   Lys Phe Leu Gly Ile Phe Asn Thr Gly Leu Lys Met Phe Pro Asp Leu
       130                 135                 140
   Thr Lys Val Tyr Ser Thr Asp Ile Phe Phe Ile Leu Glu Ile Thr Asp
   145                 150                 155                 160
   Asn Pro Tyr Met Thr Ser Ile Pro Val Asn Ala Phe Gln Gly Leu Cys
                   165                 170                 175
   Asn Glu Thr Leu Thr Leu Lys Leu Tyr Asn Asn Gly Phe Thr Ser Val
               180                 185                 190
   Gln Gly Tyr Ala Phe Asn Gly Thr Lys Leu Asp Ala Val Tyr Leu Asn
           195                 200                 205
   Lys Asn Lys Tyr Leu Thr Val Ile Asp Lys Asp Ala Phe Gly Gly Val
       210                 215                 220
   Tyr Ser Gly Pro Ser Leu Leu Asp Val Ser Gln Thr Ser Val Thr Ala
   225                 230                 235                 240
   Leu Pro Ser Lys Gly Leu Glu His Leu Lys Glu Leu Ile Ala Arg Asn
                   245                 250                 255
   Thr Trp Thr Leu Lys Glu Lys Ser His Leu Thr Pro Lys Lys Gln Gly
               260                 265                 270
   Gln Ile Ser Glu Glu Tyr Met Gln Thr Val
   275 280
<210> 5
   <211> 2615
   <212> DNA
   <213> Homo sapiens
<400> 5
   atgctgctgc tgctgctgct gctgggcctg aggctacagc tctccctggg catcatccca     60
   gttgaggagg agaacccgga cttctggaac cgcgaggcag ccgaggccct gggtgccgcc    120
   aagaagctgc agcctgcaca gacagccgcc aagaacctca tcatcttcct gggcgatggg    180
   atgggggtgt ctacggtgac agctgccagg atcctaaaag ggcagaagaa ggacaaactg    240
   gggcctgaga tacccctggc catggaccgc ttcccatatg tggctctgtc caagacatac    300
   aatgtagaca aacatgtgcc agacagtgga gccacagcca cggcctacct gtgcggggtc    360
   aagggcaact tccagaccat tggcttgagt gcagccgccc gctttaacca gtgcaacacg    420
   acacgcggca acgaggtcat ctccgtgatg aatcgggcca agaaagcagg gaagtcagtg    480
   ggagtggtaa ccaccacacg agtgcagcac gcctcgccag ccggcaccta cgcccacacg    540
   gtgaaccgca actggtactc ggacgccgac gtgcctgcct cggcccgcca ggaggggtgc    600
   aggacatcgc tacgcagctc atctccaaca tggacattga cgtgatccta ggtggaggcc    660
   gaaagtacat gtttcgcatg ggaaccccag accctgagta cccagatgac tacagccaag    720
   gtgggaccag gctggacggg aagaatctgg tgcaggaatg gctggcgaag cgccagggtg    780
   cccggtatgt gtggaaccgc actgagctca tgcaggcttc cctggacccg tctgtgaccc    840
   atctcatggg tctctttgag cctggagaca tgaaatacga gatccaccga gactccacac    900
   tggacccctc cctgatggag atgacagagg ctgccctgcg cctgctgagc aggaaccccc    960
   gcggcttctt cctcttcgtg gagggtggtc gcatcgacca tggtcatcat gaaagcaggg   1020
   cttaccgggc actgactgag acgatcatgt tcgacgacgc cattgagagg gcgggccagc   1080
   tcaccagcga ggaggacacg ctgagcctcg tcactgccga ccactcccac gtcttctcct   1140
   tcggaggcta ccccctgcga gggagctcca tcttcgggct ggcccctggc aaggcccggg   1200
   acaggaaggc ctacacggtc ctcctatacg gaaacggtcc aggctatgtg ctcaaggacg   1260
   gcgcccggcc ggatgttacc gagagcgaga gcgggagccc cgagtatcgg cagcagtcag   1320
   cagtgcccct ggacgaagag acccacgcag gcgaggacgt ggcggtgttc gcgcgcggcc   1380
   cgcaggcgca cctggttcac ggcgtgcagg agcagacctt catagcgcac gtcatggcct   1440
   tcgccgcctg cctggagccc tacaccgcct gcgacctggc gccccccgcc ggcaccaccg   1500
   acgccgcgca cccgggttac tctagagtcg ggcggccggg ccgcttcgag cagacaaagc   1560
   cttcacagct gcagtcccga gagctgtcag ggtcgcgctg ccccgagccc tgcgactgcg   1620
   caccggatgg cgccctgcgc tgtcctggcc ctcgagccgg cctcgccaga ctatctctca   1680
   cctatctccc tgtcaaagta attccatcac aagctttcag gggacttaat gaggtcgtaa    1740
   aaattgaaat ctctcagagt gattccctgg aaaggataga agctaatgcc tttgacaacc 1800
   tcctcaattt gtctgaacta ctgatccaga acaccaaaaa cctgctatac attgaacctg 1860
   gtgcttttac aaacctccct cggttaaaat acctgagcat ctgtaacaca ggcatccgaa 1920
   cccttccaga tgttacgaag atctcctcct ctgaatttaa tttcattctg gaaatctgtg 1980
   ataacttaca cataacctca atccctgtga atgcttttca gggactatgc aatgaaacct 2040
   tgacactgaa gctgtacaac aacggcttta cttcagtcca aggatatgct ttcaatggga 2100
   caaagctgga tgctgtttac ctaaacaaga ataaatacct gacagttatt gacaaagatg 2160
   catttggagg agtatacagt ggaccaagct tgctggacgt gtctcaaacc agtgtcactg 2220
   cccttccatc caaaggcctg gagcacctga aggaactgat agcaagaaac acctggactc 2280
   ttaagaaact tccactttcc ttgagtttcc ttcacctcac acgggctgac ctttcttacc 2340
   caagccactg ctgtgccttt aagaatcaga agaaaatcag aggaatcctt gagtccttga 2400
   tgtgtaatga gagcagtatg cagagcttgc gccagagaaa atctgtgaat gccttgaata 2460
   gccccctcca ccaggaatat gaagagaatc tgggtgacag cattgttggg tacaaggaaa 2520
   agtccaagtt ccaggatact cataacaacg ctcattatta cgtcttcttt gaagaacaag 2580
   aggatgagat cattggtttt ggccaggagc tctaa 2615
<210> 6
   <211> 871
   <212> PRT
   <213> Homo sapiens
<400> 6
<210> 7
   <211> 1179
   <212> DNA
   <213> Homo sapiens
<400> 7
   atgaggccgg cggacttgct gcagctgcag ctgctggtgc tggcagtgct gctgctgaag      60
   ccttcacagc tgcagtcccg agagctgtca gggtcgcgct gccccgagcc ctgcgactgc     120
   gcaccggatg gcgccctgcg ctgtcctggc cctcgagccg gcctcgccag actatctctc     180
   acctatctcc ctgtcaaagt aattccatca caagctttca ggggacttaa tgaggtcgta     240
   aaaattgaaa tctctcagag tgattccctg gaaaggatag aagctaatgc ctttgacaac     300
   ctcctcaatt tgtctgaact actgatccag aacaccaaaa acctgctata cattgaacct     360
   ggtgctttta caaacctccc tcggttaaaa tacctgagca tctgtaacac aggcatccga     420
   acccttccag atgttacgaa gatctcctcc tctgaattta atttcattct ggaaatctgt     480
   gataacttac acataacctc aatccctgtg aatgcttttc agggactatg caatgaaacc     540
   ttgacactga agctgtacaa caacggcttt acttcagtcc aaggatatgc tttcaatggg     600
   acaaagctgg atgctgttta cctaaacaag aataaatacc tgacagttat tgacaaagat     660
   gcatttggag gagtatacag tggaccaagc ttgctggacg tgtctcaaac cagtgtcact     720
   gcccttccat ccaaaggcct ggagcacctg aaggaactga tagcaagaaa cacctggact     780
   cttaagaaac ttccactttc cttgagtttc cttcacctca cacgggctga cctttcttac     840
   ccaagccact gctgtgcctt taagaatcag aagaaaatca gaggaatcct tgagtccttg     900
   atgtgtaatg agagcagtat gcagagcttg cgccagagaa aatctgtgaa tgccttgaat     960
   agccccctcc accaggaata tgaagagaat ctgggtgaca gcattgttgg gtacaaggaa   1020
   aagtccaagt tccaggatac tcataacaac gctcattatt acgtcttctt tgaagaacaa    1080
   gaggatgaga tcattggttt tggccaggag ctcgaaaaat cccatctaac cccaaagaag    1140
   caaggccaaa tctcagaaga gtatatgcaa acggtttaa                           1179
<210> 8
   <211> 392
   <212> PRT
   <213> Homo sapiens
<400> 8
   Met Arg Pro Ala Asp Leu Leu Gln Leu Gln Leu Leu Val Leu Ala Val
   1 5 10 15
   Leu Leu Leu Lys Pro Ser Gln Leu Gln Ser Arg Glu Leu Ser Gly Ser
   20 25 30
   Arg Cys Pro Glu Pro Cys Asp Cys Ala Pro Asp Gly Ala Leu Arg Cys
   35 40 45
   Pro Gly Pro Arg Ala Gly Leu Ala Arg Leu Ser Leu Thr Tyr Leu Pro
   50 55 60
   Val Lys Val Ile Pro Ser Gln Ala Phe Arg Gly Leu Asn Glu Val Val
   65 70 75 80
   Lys Ile Glu Ile Ser Gln Ser Asp Ser Leu Glu Arg Ile Glu Ala Asn
   85 90 95
   Ala Phe Asp Asn Leu Leu Asn Leu Ser Glu Leu Leu Ile Gln Asn Thr
   100 105 110
   Lys Asn Leu Leu Tyr Ile Glu Pro Gly Ala Phe Thr Asn Leu Pro Arg
   115 120 125
   Leu Lys Tyr Leu Ser Ile Cys Asn Thr Gly Ile Arg Thr Leu Pro Asp
   130 135 140
   Val Thr Lys Ile Ser Ser Ser Glu Phe Asn Phe Ile Leu Glu Ile Cys
   145 150 155 160
   Asp Asn Leu His Ile Thr Ser Ile Pro Val Asn Ala Phe Gln Gly Leu
   165 170 175
   Cys Asn Glu Thr Leu Thr Leu Lys Leu Tyr Asn Asn Gly Phe Thr Ser
   180 185 190
   Val Gln Gly Tyr Ala Phe Asn Gly Thr Lys Leu Asp Ala Val Tyr Leu
   195 200 205
   Asn Lys Asn Lys Tyr Leu Thr Val Ile Asp Lys Asp Ala Phe Gly Gly
   210 215 220
   Val Tyr Ser Gly Pro Ser Leu Leu Asp Val Ser Gln Thr Ser Val Thr
   225 230 235 240
   Ala Leu Pro Ser Lys Gly Leu Glu His Leu Lys Glu Leu Ile Ala Arg
   245 250 255
   Asn Thr Trp Thr Leu Lys Lys Leu Pro Leu Ser Leu Ser Phe Leu His
   260 265 270
   Leu Thr Arg Ala Asp Leu Ser Tyr Pro Ser His Cys Cys Ala Phe Lys
   275 280 285
   Asn Gln Lys Lys Ile Arg Gly Ile Leu Glu Ser Leu Met Cys Asn Glu
   290 295 300
   Ser Ser Met Gln Ser Leu Arg Gln Arg Lys Ser Val Asn Ala Leu Asn
   305 310 315 320
   Ser Pro Leu His Gln Glu Tyr Glu Glu Asn Leu Gly Asp Ser Ile Val
   325 330 335
   Gly Tyr Lys Glu Lys Ser Lys Phe Gln Asp Thr His Asn Asn Ala His
   340 345 350
   Tyr Tyr Val Phe Phe Glu Glu Gln Glu Asp Glu Ile Ile Gly Phe Gly
   355 360 365
   Gln Glu Leu Glu Lys Ser His Leu Thr Pro Lys Lys Gln Gly Gln Ile
   370 375 380
   Ser Glu Glu Tyr Met Gln Thr Val
   385 390
<210> 9
   <211> 1323
   <212> DNA
   <213> Homo sapiens
<400> 9
   atgggagtca aagttctgtt tgccctgatc tgcatcgctg tggccgaggc caagcccacc 60
   gagaacaacg aagacttcaa catcgtggcc gtggccagca acttcgcgac cacggatctc 120
   gatgctgacc gcgggaagtt gcccggcaag aagctgccgc tggaggtgct caaagagatg 180
   gaagccaatg cccggaaagc tggctgcacc aggggctgtc tgatctgcct gtcccacatc 240
   aagtgcacgc ccaagatgaa gaagttcatc ccaggacgct gccacaccta cgaaggcgac 300
   aaagagtccg cacagggcgg cataggcgag gcgatcgtcg acattcctga gattcctggg 360
   ttcaaggact tggagcccat ggagcagttc atcgcacagg tcgatctgtg tgtggactgc 420
   acaactggct gcctcaaagg gcttgccaac gtgcagtgtt ctgacctgct caagaagtgg 480
   ctgccgcaac gctgtgcgac ctttgccagc aagatccagg gccaggtgga caagatcaag 540
   ggggccggtg gtgacagcat cggaatgggg tgttcgtctc caccctgcga gtgccatcag 600
   gaggaggact tcagagtcac ctgcaaggat attcaacgca tccccagctt accgcccagt 660
   acgcagactc tgaagcttat tgagactcac ctgagaacta ttccaagtca tgcattttct 720
   aatctgccca atatttccag aatctacgta tctatagatg tgactctgca gcagctggaa 780
   tcacactcct tctacaattt gagtaaagtg actcacatag aaattcggaa taccaggaac 840
   ttaacttaca tagaccctga tgccctcaaa gagctccccc tcctaaagtt ccttggcatt 900
   ttcaacactg gacttaaaat gttccctgac ctgaccaaag tttattccac tgatatattc 960
   tttatacttg aaattacaga caacccttac atgacgtcaa tccctgtgaa tgcttttcag 1020
   ggactatgca atgaaacctt gacactgaag ctgtacaaca atggctttac ttcagtccaa 1080
   ggatatgctt tcaatgggac aaagctggat gctgtttacc taaacaagaa taaatacctg 1140
   acagttattg acaaagatgc atttggagga gtatacagtg gaccaagctt gctggacgtg 1200
   tctcaaacca gtgtcactgc ccttccatcc aaaggcctgg agcacctgaa ggaactgata 1260
   gcaagaaaca cctggactct taagaaaaca ctgccctcca aacatcacca tcaccatcac 1320
   taa 1323
<210> 10
   <211> 440
   <212> PRT
   <213> Homo sapiens
<400> 10
   Met Gly Val Lys Val Leu Phe Ala Leu Ile Cys Ile Ala Val Ala Glu
   1               5                   10                  15
   Ala Lys Pro Thr Glu Asn Asn Glu Asp Phe Asn Ile Val Ala Val Ala
               20                  25                  30
   Ser Asn Phe Ala Thr Thr Asp Leu Asp Ala Asp Arg Gly Lys Leu Pro
           35                  40                  45
   Gly Lys Lys Leu Pro Leu Glu Val Leu Lys Glu Met Glu Ala Asn Ala
       50                  55                  60
   Arg Lys Ala Gly Cys Thr Arg Gly Cys Leu Ile Cys Leu Ser His Ile
   65                  70                  75                  80
   Lys Cys Thr Pro Lys Met Lys Lys Phe Ile Pro Gly Arg Cys His Thr
                   85                  90                  95
   Tyr Glu Gly Asp Lys Glu Ser Ala Gln Gly Gly Ile Gly Glu Ala Ile
               100                 105                 110
   Val Asp Ile Pro Glu Ile Pro Gly Phe Lys Asp Leu Glu Pro Met Glu
           115                 120                 125
   Gln Phe Ile Ala Gln Val Asp Leu Cys Val Asp Cys Thr Thr Gly Cys
       130                 135                 140
   Leu Lys Gly Leu Ala Asn Val Gln Cys Ser Asp Leu Leu Lys Lys Trp
   145                 150                 155                 160
   Leu Pro Gln Arg Cys Ala Thr Phe Ala Ser Lys Ile Gln Gly Gln Val
                   165                 170                 175
   Asp Lys Ile Lys Gly Ala Gly Gly Asp Ser Ile Gly Met Gly Cys Ser
               180                 185                 190
   Ser Pro Pro Cys Glu Cys His Gln Glu Glu Asp Phe Arg Val Thr Cys
           195                 200                 205
   Lys Asp Ile Gln Arg Ile Pro Ser Leu Pro Pro Ser Thr Gln Thr Leu
       210                 215                 220
   Lys Leu Ile Glu Thr His Leu Arg Thr Ile Pro Ser His Ala Phe Ser
   225                 230                 235                 240
   Asn Leu Pro Asn Ile Ser Arg Ile Tyr Val Ser Ile Asp Val Thr Leu
                   245                 250                 255
   Gln Gln Leu Glu Ser His Ser Phe Tyr Asn Leu Ser Lys Val Thr His
               260                 265                 270
   Ile Glu Ile Arg Asn Thr Arg Asn Leu Thr Tyr Ile Asp Pro Asp Ala
           275                 280                 285
   Leu Lys Glu Leu Pro Leu Leu Lys Phe Leu Gly Ile Phe Asn Thr Gly
       290                 295                 300
   Leu Lys Met Phe Pro Asp Leu Thr Lys Val Tyr Ser Thr Asp Ile Phe
   305                 310                 315                 320
   Phe Ile Leu Glu Ile Thr Asp Asn Pro Tyr Met Thr Ser Ile Pro Val
                   325                 330                 335
   Asn Ala Phe Gln Gly Leu Cys Asn Glu Thr Leu Thr Leu Lys Leu Tyr
               340                 345                 350
   Asn Asn Gly Phe Thr Ser Val Gln Gly Tyr Ala Phe Asn Gly Thr Lys
           355                 360                 365
   Leu Asp Ala Val Tyr Leu Asn Lys Asn Lys Tyr Leu Thr Val Ile Asp
       370                 375                 380
   Lys Asp Ala Phe Gly Gly Val Tyr Ser Gly Pro Ser Leu Leu Asp Val
   385                 390                 395                 400
   Ser Gln Thr Ser Val Thr Ala Leu Pro Ser Lys Gly Leu Glu His Leu
                   405                 410                 415
   Lys Glu Leu Ile Ala Arg Asn Thr Trp Thr Leu Lys Lys Thr Leu Pro
               420                 425                 430
   Ser Lys His His His His His His
           435                 440

## Beschreibung

### TECHNISCHES GEBIET DER ERFINDUNG

Die Erfindung betrifft Verfahren zur Bestimmung unterschiedlicher Typen von Autoantikörpern gegen den Rezeptor für das Thyroid stimulierende Hormon (TSH-Rezeptor) mit hoher Spezifität und in diesem Verfahren als Bindungsreagenz verwendbare neue TSH-Rezeptorchimären.

### TECHNOLOGISCHER HINTERGRUND DER ERFINDUNG

Der TSH-Rezeptor (TSH-R) spielt eine Schlüsselrolle für die Funktion und das Wachstum von Schilddrüsenzellen. Dieser Rezeptor ist ein Glied einer Unterfamilie der G-Protein gekoppelten Glycoproteinrezeptoren, die außerdem insbesondere noch die Rezeptoren für das luteinisierende Hormon/Choriongonadotropin (LH/CGR) und das Follikel stimulierende Hormon (FSHR) umfasst. Die Rezeptoren dieser Unterfamilie weisen eine große N-terminale extrazelluläre Domäne auf, die für die Ligandenbindung von essentieller Bedeutung ist und für die gezeigt wurde, dass sie an der Signalübermittlung beteiligt ist. Die Übermittlung des TSHR-Signals wird überwiegend durch Aktivierung von Adenylatzyklase vermittelt, was zu einer Erhöhung des intrazellulären cAMP-Niveaus führt.

Ein Teil des großen Interesses an dem TSH-Rezeptor ist auf seine Rolle als primäres Autoantigen bei Schilddrüsen-Autoimmunkrankheiten zurückzuführen, die vom Auftreten von Autoantikörpern gegen den TSH-Rezeptor begleitet sind. Zu derartigen Schilddrüsen-Autoimmunerkrankungen gehören insbesondere die Basedowsche Krankheit, eine zu Schilddrüsenüberfunktion führende Autoimmunerkrankung, die zu den häufigsten menschlichen Autoimmunerkrankungen überhaupt gehört. Die Basedowsche Krankheit wird durch Aktivierung der Adenylatcyclase und resultierenden cAMP-Anstieg verursacht. Dadurch kommt es zur SchilddrüsenÜberfunktion, Kropfbildung und gegebenenfalls Aügenveränderungen. Die Autoantikörper gegen den TSH-Rezeptor können auch blockierenden Charakter haben und somit die Adenylatcyclase und das cAMP hemmen. In diesem Falle kommt es zu einer Unterfunktion der Schilddrüse. Ein gleichzeitiges Auftreten von stimulierenden und blockierenden Autoantikörpern bei den betroffenen Patienten ist ebenfalls möglich, wobei der Anteil der stimulierenden Antikörper in der Regel überwiegt.

Zum Nachweis solcher Autoimmunantikörper gibt es seit einiger Zeit einen Bioassay, in dem die cAMP-Zunahme gemessen wird. Dieses Messverfahren ist sehr zeitaufwendig. Darüber hinaus ist der Bioassay nicht zuverlässig, da er falsch positive Ergebnisse liefern kann. Im Rahmen dieser Beschreibung wird diese Art von Messverfahren als Bioassay zur Abgrenzung von den in-vitro-Verfahren zur Bestimmung von Autoimmunantikörpern gegen den TSH-Rezeptor bezeichnet. Bei einem auf dem Markt befindlichen in-vitro-Nachweisverfahren für Autoimmunantikörper wird ein aus Schweineschilddrüsenmembran extrahierter TSH-Rezeptor eingesetzt (erste Generation der in-vitro-Verfahren). In einem anderen Test zum Nachweis von Autoimmunantikörpern gegen den TSH-Rezeptor wird ein humanes vollständiges rekombinantes TSH-Rezeptorprotein (Wildtyp) in einem Kompetitionstest eingesetzt (zweite Generation der in-vitro-Verfahren).

Thyroid Vol.7 (1997) 867-877 beschreibt die Epitope für stimulierende und blockierende Antikörper am TSH-Rezeptor. Die Mehrzahl der funktionellen Epitope für stimulierende Antikörper sind im Bereich der Aminosäuren 8 bis 168, diejenigen für die blockierenden Antikörper im bereich der Aminosäuren 261 bis 370 des Rezeptorproteins. Für Aktivitätsmessungen wird der zuvor genannte Bioassay eingesetzt.

Durch die WO 01/27634 A1 wird erstmalig ein quantitatives Verfahren zum Nachweis von Autoimmunantikörpern unterschiedlicher Spezifität nebeneinander bereitgestellt, das schnell reproduzierbar und mit hoher Genauigkeit durchführbar ist. Zu diesem Zweck werden TSH-Rezeptorchimären eingesetzt, die sich von dem Wildtyp-Rezeptor dadurch unterscheiden, dass einzelne Sequenzen, an denen Autoimmunantikörper binden, ausgetauscht sind durch entsprechende Sequenzen eines anderen Rezeptors aus der Klasse der G-Protein gekoppelten Rezeptoren. Die TSH-Rezeptorchimären basieren auf dem vollständigen TSH-Rezptorprotein. Allerdings ist der Messaufwand hoch. Das Trennen durch Zentrifugieren macht das Verfahren zu umständlich für eine routinemäßige Nutzung. Auch muss der Test im Eisbad oder bei 4°C durchgeführt werden, da die TSH-Rezeptorchimären nicht sehr stabil sind. Eine entsprechende technische Lehre findet sich in der WO O1/63295 A1, in der die Verwendung einer Sandwich-Technik zur Detektion vorgeschlagen wird. Es hat sich jedoch herausgestellt, dass in der Regel mit den dort vorgeschlagenen Testmaterialien, die unspezifische Bindung zu hoch ist. Ein Test zum Nachweis von Autoimmunantikörpern auf der Grundlage der Erkenntnisse der beiden zuvor genannten Patentanmeldungen ist auf dem Markt nicht erhältlich.

Ein seit einiger Zeit auf dem Markt befindliches Messverfahren zum Nachweis von Autoimmunantikörpern, das den vollständigen unveränderten TSH-Rezeptor in einem Kompetitionsassay einsetzt, wird für die Zwecke dieser Beschreibung als Nachweisverfahren der zweiten Generation bezeichnet. Dieser Test eignet sich zum Nachweis von Morbus Basedow. Nachteilig hingegen ist, dass stimulierende blockierende und neutrale Autoimmunantikörper nicht zu unterscheiden sind. Ferner werden nicht alle Subtypen erfasst, da das verdrängte TSH nur zu 30 bis 40 % an das Epitop für stimulierende und blockierende Autoantikörper bindet.

### STAND DER TECHNIK

Die DE 196 45 729 C1 beschreibt einen Test zum Nachweis von gegen den TSH-Rezeptor gebildeten Autoimmunantikörpern unter Verwendung eines TSH-Rezeptorpräparats, bei dem der Rezeptor um einen signalgebendes Peptid verlängert wurde. Hier werden keine Rezeptorchimären verwendet, bei denen die bindenden Epitope des Rezeptors durch Peptidreste eines anderen verwandten Rezeptors ersetzt worden sind.

Die WO 00/00590 A1 beschreibt einen C-terminal trunkierten TSH-Rezptor und beschreibt in allgemeiner Weise dessen Eignung zur Diagnose und Therapie.

### ZUSAMMENFASSUNG DER ERFINDUNG

Ausgehend von diesem Stand der Technik liegt der Erfindung die Aufgabe zu Grunde, das aus der WO 01/27634 A1 bekannte Verfahren zu modifizieren, um die Genauigkeit und Aussagekraft dieses Tests noch weiter zu erhöhen und ein solches Verfahren für Automaten nutzbar zu machen.

Gelöst wird diese Aufgabe durch ein Anspruch 1 der Patentansprüche.

### BESCHREIBUNG DER BEVORZUGTEN AUSFÜHRUNGSFORMEN

- Abb. 1: zeigt schematisch eine erfindungsgemäße Verfahrensweise.
- Abb. 2: zeigt schematisch eine weitere erfindungsgemäße Verfahrensweise.
- Abb. 3: zeigt schematisch eine weitere erfindungsgemäße Verfahrensweise.
- Abb. 4: zeigt schematisch eine nicht erfindungsgemäße Verfahrensweise.

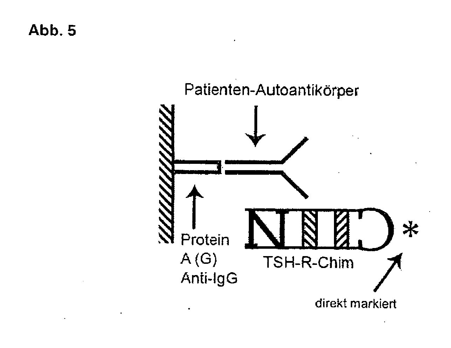

- Abb. 5: zeigt schematisch eine nicht erfindungsgemäße Verfahrensweise.
- Abb. 6: zeigt eine nach dem erfindungsgemäßen Verfahren erhaltene Standardkurve mit NIBSC (WHO)-Standardlösungen vcn TSH-Autoimmunantikörpern.

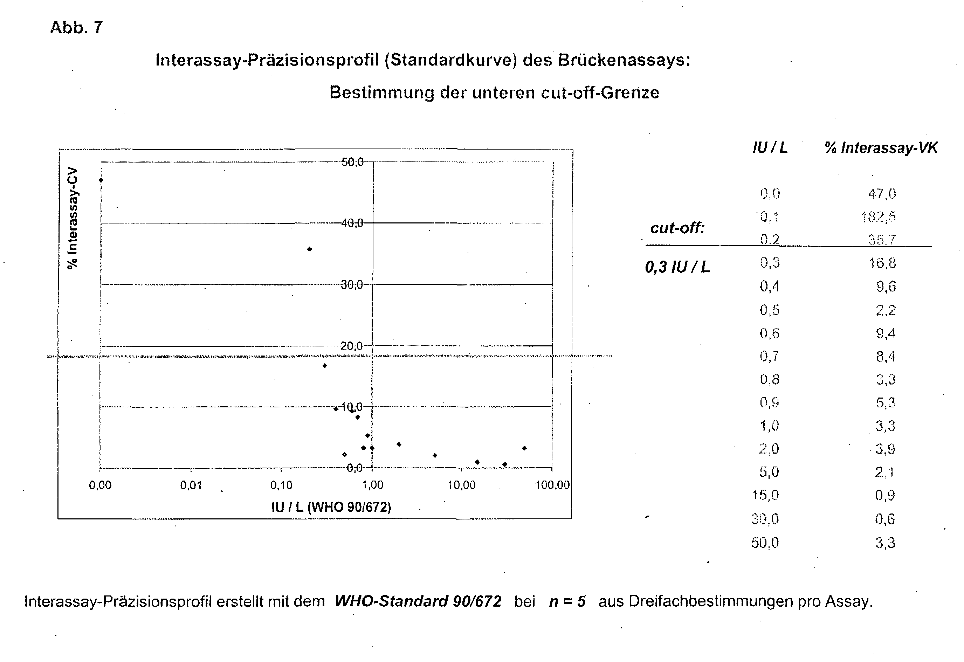

- Abb. 7: zeigt ein Interassay-Präzisionsprofil, erstellt mit dem WHO-Standard 90/672 bei n=5 aus Dreifachbestimmungen pro Test.
- Abb. 8: zeigt einen Vergleichs der Messergebnisse, die an Patientenseren mit dem erfindungsgemäßen Verfahren und mit einem Test der so genannten zweiten Generation erzielt wurden.

Sequenz Nr. 1 zeigt die Nukleotidsequenz einer trunkierten TSH-Rezeptorchimäre B (Extrazelluläranteil) fusioniert mit der sekretorischen alkalischen Phosphatase.

Sequenz Nr. 2 zeigt die Aminosäuresequenz einer trunkierten TSH-Rezeptorchimäre B (Extrazelluläranteil) fusioniert der sekretorischen alkalischen Phosphatase.

Sequenz Nr. 3 zeigt die Nukleotidsequenz eines Fusionsproteins aus einer trunkierten TSH-Rezeptorchimäre B (Extrazelluläranteil) fusioniert mit einem immunogenen Epitop.

Sequenz Nr. 4 zeigt die Aminosäuresequenz eines Fusionsproteins aus einer trunkierten TSH-Rezeptorchimäre B (Extrazelluläranteil) fusioniert mit einem immunogenen Epitop.

Sequenz Nr. 5 zeigt die Nukleotidsequenz eines Fusionsproteins aus einer trunkierten TSH-Rezeptorchimäre A (Extrazelluläranteil) fusioniert mit dem sekretorischen Signalpeptid der sekretorischen alkalischen Phosphatase (SEAP) und der SEAP.

Sequenz Nr. 6 zeigt die Aminosäuresequenz eines Fusionsproteins aus einer trunkierten TSH-Rezeptorchimäre A (Extrazelluläranteil) fusioniert mit dem sekretorischen Signalpeptid der SEAP und der SEAP.

Sequenz Nr. 7 zeigt die Nukleotidsequenz eines Fusionsproteins aus einer trunkierten TSH-Rezeptorchimäre A (Extrazelluläranteil) fusioniert mit einem hoch immunogenen Epitop für einen polyklonalen Antikörper.

Sequenz Nr. 8 zeigt die Aminosäuresequenz eines Fusionsproteins aus einer trunkierten TSH-Rezeptorchimäre A (Extrazelluläranteil) fusioniert mit einem hochimmunogenen Epitop für einen polyklonalen Antikörper.

Sequenz Nr. 9 zeigt die Nukleotidsequenz eines Fusionsproteins aus einer trunkierten TSH-Rezeptorchimäre 3 (Extrazelluläranteil) fusioniert mit einer gLUC-Signalpeptidsequenz und einer Gausia Luciferasesequenz.

Sequenz Nr. 10 zeigt die Aminosäuresequenz eines Fusionsproteins aus einer trunkierten TSH-Rezeptorchimäre B (Extrazelluläranteil) fusioniert mit einer gLUC-Signalpeptidsequenz und einer Gausia Luciferasesequenz.

Die-erfindungsgemäß eingesetzten TSH-Rezeptorchimären sind solche, bei denen Teile der Aminosäuresequenz durch vergleichbare Sequenzen eines anderen Rezeptors mit einem anderen Bindungsverhalten gegenüber TSH-Rezeptorautoimmunantikörpern, insbesondere nicht bindende Sequenzen, ersetzt sind. Solche vergleichbaren Sequenzen können beispielsweise Sequenzen eines Ratten LG-CG-Rezeptors sein. Ausgetauscht wurden bei den erfindungsgemäß eingesetzten Rezeptorchimären folglich die Epitope, an denen stimulierende und/oder blockierende Autoimmunantikörper binden. Bei den TSH-Rezeptorchimären, an denen neutrale Autoimmunantikörper binden, wurden folglich die Epitope für stimulierende und für blockierende Autoimmunantikörper ausgetauscht. Diese Rezepterchimären können gemäß Biochem. Biophys. Res. Comun. (1991), 179:70-77 oder WO 01/27634 konstruiert werden. Auf beide Dokumente wird für die Zwecke der Offenbarung dieser Erfindung Bezug genommen.

Erfindungsgemäß werden die TSH-Rezeptöchimären A, B und C unterschieden. Bei der Chimäre A können vorzugsweise die TSH-Rezeptoraminosäuren 8 bis 165 durch im Wesentlichen die vergleichbaren Aminosäuren 10 bis 166 des LH-CGR ersetzt sein. Bei der Chimäre B können vorzugsweise die TSH-Rezeptoraminosäuren 261 bis 370 durch im Wesentlichen die entsprechenden Aminosäuren 261 bis 329 eines Ratten-LH-CGR ersetzt sein. Bei der Chimäre C können sowohl die Aminosäuren 8 bis 165 als auch die Aminosäuren 261 bis 370 des TSH-Rezeptors durch entsprechende LH-CGR-Aminosäuren ersetzt sein.

Mit der TSH-Rezeptorchimäre A werden blockierende Autoimmunantikörper, mit der Chimäre B stimulierende Autoimmunantikörper und mit der Chimäre C neutrale Autoimmunantikörper aus einer Patientenprobe nachgewiesen.

Der TSH-Rezeptor erstreckt sich vom Cytosol durch die Zellmembran in den Extrazellulärraum. Der extrazelluläre Anteil des Rezeptorproteins weist die Bindungsstellen für TSH und die Autoimmunantikörper auf. überraschen wurde festgestellt, dass ein trunkiertes TSH-Rezeptorpeptid, welches im Wesentlichen nur den extrazelluären Anteil des TSH-Rezptorproteins aufweist stabil gelagert werden kann, ohne dass die Fähigkeit zur Bindung von TSH verloren geht. Dasselbe wurde auch für die Fähigkeit zur Bindung von Autoimmunantikörpern erkannt.

Der extrazelluläre Anteil der TSH-Rezeptorchimäre kann im Wesentlichen ein Peptid von 1 bis 418 Aminosäuren umfassen. Das entspricht den Aminosäuren 1 bis 418 des extrazellulären Teils des Wildtyp-TSH-Rezeptors. Den eingesetzten trunkierten TSH-Rezeptorchimären A, B und C fehlt es somit im Wesentlichen an dem Cytosol- und dem Membrananteil der bekannten TSH-Rezeptorchimären. Die trunkierten TSH-Rezeptorchimären lassen sich wesentlich einfacher als die bisher bekannten vollständigen TSH-Rezeptorchimären in dem erfindungsgemäßen Nachweisverfahren einsetzen.

Die eingesetzten erfindungsgemäßen trunkierten TSH-Rezetorchimären A, B und C sind solche, denen der Cytosolanteil und im wesentlichen der Membrananteil des TSH-Wildtyprezeptors fehlt. Der Extrazelluläranteil des Wildtyp-TSH-Rezeptors ist in diesen Fällen als Chimäre A, B oder C, wie zuvor beschrieben, ausgebildet.

Besonders vorteilhaft an diesen trunkierten TSH-Rezeptorchimären ist, dass bei deren Produktion in rekombinierten Zellen, die trunkierten TSH-Rezeptorchimären A, B und C in den Extrazellulärraum sezerniert werden, wenn Signalpeptide bzw. die entsprechende Nukleotidsequenz von beispielsweise der alkalischen Phosphatase oder des Transthyretins vor die TSH-Rezeptorchimär-Nukleotidse-quenz insertiert sind. Es ist dann kein Aufschluss der Zellen zur Gerinnung der TSH-Rezeptorchimären A, B oder C erforderlich. Besonders bevorzugt ist das Signalpeptid des Enzyms Transthyretin. Unter Signalpeptid wird in dieser Erfindungsbeschreibung ein Peptidrest verstanden, der wenigsten die für die Sekretion der trunkierten Rezeptorchimären aus der Zelle erforderlichen Aminosäuren aufweist.

Gemäß dieser Anmeldung ist das Signalpeptid Bestandteil der TSH-Rezptorchimären und zusätzlich kann ein für Detektionszwecke nutzbares Enzym mit seiner sekretorischen Signalpeptidsequenz in der TSH-Rezeptorchimäre enthalten sein.

In einer Ausführungsform der Erfindung kann die erste, an eine Festphase gebundene TSH-Rezeptorchimäre an ihrem C-terminalen Ende fusioniert sein mit einem Peptid. Ein solches Peptid ist beispielsweise eine hochimmunogene Teilsequenz des Peptids Thyrostimulin oder aus dem C-Terminus des TSH-Rezeptors. In diesem Fall ist der die erste TSH-Rezeptorchimäre an der Festphase immobilisierende Antikörper ein gegen dieses Peptid gerichteter monoklonaler oder polyklonaler Antikörper, beispielsweise ein gegen Thyrostimulin gerichteter Antikörper.

Durch die erfindungsgemäße Verfahrensweise werden Probleme wie unspezifische Bindungen, die bei einem Brückenassay auftreten können, unterdrückt. Der Einsatz dieser zweiten derart modifizierten Rezeptorchimäre dient der weiteren Erhöhung der Spezifität und der Sensitivität des Verfahrens.

Gemäß einer weiteren Ausführungsform kann eine modifizierte zweite TSH-Rezeptorchimäre an ihrem C-terminalen Ende mit einem hochimmunogenem Peptid fusioniert sein. Ein solches kann ausgewählt sein aus einer Teilsequenz von Thyrostimulin oder dem C-terminalen Ende des TSH-Rezeptors. Eine solche Teilsequenz kann dem cytosolischen Teil des TSH-Rezeptors entnommen sein, beispielsweise codiert durch die Nukleotide 743 bis 763 des TSH-Rezeptors. Auch eine derart erhaltene zweite TSH-Rezeptorchimäre weist den Vorteil auf, dass ein Binden der zweiten TSH-Rezeptorchimäre an dem immobilisierten Antikörper, an dem bereits eine erste TSH-Rezeptorchimäre C-terminal gebunden hat, vermieden wird. Durch die Präsentation eines hochimmunogenen Peptidrests kann ein zweiter markierter Antikörper einfach und spezifisch gebunden werden. Durch die Fusion mit einer Teilsequenz des Peptids Thyrostimulin ergibt sich derselbe Vorteil. Antikörper gegen hochimmunogene Peptidsequenzen weisen eine hohe Bindungsaffinität auf. Folglich wird eine hohe Spezifität der Bindung erreicht, wohingegen unspezifische Bindungen weitgehend verringert werden.

Die erfindungsgemäß eingesetzte erste oder zweite TSH-Rezeptorchimäre kann zur Detektion modifiziert sein. Diese Modifizierung kann eine Markierung zur Detektion sein oder eine Markierung durch eine immunogene Peptidsequenz, die durch einen zur Detektion geeigneten sekundären Antikörper erkannt wird.

In dem erfindungsgemäßen Verfahren sind die so genannten ersten TSH-Rezeptorchimären A, B und C an einer festen Phase gebunden. Die Bindung der TSH-Rezeptorchimären an der festen Phase kann in diesem Fall über einen immobilisierenden Antikörper erfolgen, der beispielsweise gegen ein C-terminales Epitop der TSH-Rezeptorchimären gerichtet ist. Ein solcher Antikörper kann ein polyklonaler oder monoklonaler Antikörper sein.

Zum Nachweis des Bindens eines Autoimmunantikörpers an einer der TSH-Rezeptorchimären A, B oder C kann ein sekundärer Antikörper eingesetzt werden. Der sekundäre Antikörper kann zusätzlich zu dem immobilisierenden Antikörper vorhanden sein. Er kann ein monoklonaler oder ein polyklonaler Antikörper sein.

Die eingesetzten TSH-Rezeptorchimären, erste oder zweite TSH-Rezeptorchimäre, können je nach gewünschtem Assaydesign markiert sein. Eine direkte Markierung ist eine Markierung der TSH-Rezptorchimären. Eine indirekte Markierung ist der Einsatz eines sekundären markierten Antikörpers. Die Markierungen können derart sein, dass sie unmittelbar oder mittelbar ein nachweisbares Signal liefern.

Markierungsmittel können durch Fusion oder chemische Bindung mit den TSH-Rezeptorchimären oder den sekundären Antikörpern verbunden sein. Vorzugsweise sind die Markierungsmittel N-terminal zu der TSH-Rezptorchimären mit dieser fusioniert.

Geeignete Markierungen erfolgen beispielsweise durch Enzyme wie alkalische Phosphatase (AP), sekretorische alkalische Phosphatase (SEAP), Leuchtkäfer-Luciferase und Peroxidase oder einen Farbstoff wie einen Acridinfarbstoff, ein fluoreszierendes oder bio-/chemieluminiszierendes Material. Im Falle der zuvor genannten Enzyme ist die dafür codierende Nukleotidsequenz vorzugsweise mit der Nukleotidsequenz der TSH-Rezeptorchimären fusioniert. Geeignete Markierungen erfolgen beispielsweise ferner durch FITC, Biotinylierung und Streptavidin.

Geeignete feste Phasen umfassen Kunststoffkörper, wie Kunststoffröhrchen, Kunststoffplättchen und magnetische und nichtmagnetische Kunststoffpartikel. Für die erfindungsgemäß eingesetzten festen Phasen geeignete Kunststoffe sind solche, die das Binden von Proteinen durch chemische oder physikalische Reaktion ermöglichen. Diese umfassen Kügelchen, Mikrotiterplatten und Tubes, welche aus Polystyrol, Polyethylen oder anderen bekannten polymeren Materialien bestehen können. Solche feste Phasen sind dem Fachmann bekannt und im Handel erhältlich.

Die erfindungsgemäßen trunkierten TSH-Rezeptorchimären können lyophilisiert gelagert werden. Sie können über einen monoklonalen oder polyklonalen Antikörper an eine Festphase gebunden in lyophilisierter Form gelagert werden. Für die Nachweisreaktion erfolgt eine Rekonstitution der lyophilisierten Komponenten durch Lösen in einem Assaypuffer.

Die erfindungsgemäßen trunkierten TSH-Rezeptorchimären besitzen in gelöster Form eine hohe Stabilität. Sie bleiben bei 4°C 4 bis 7 Tage, bei 24°C 3 bis 6 Tage und bei 37°C 24 Stunden lang stabil. Diese Bedingungen eignen sich für die Durchführung des erfindungsgemäßen Nachweisverfahrens auch auf Automaten, auf denen die Komponenten bei 4°C gelagert werden, während die Versuchsreaktion problemlos bei 37°C ablaufen kann. Demgegenüber bleibt die vollständige TSH-Rezeptorchimäre bei 4°C 3 bis 6 Tage, bei 24°C 24 bis 48 Stunden und bei 37°C nur 3 Stunden lang stabil.

Beider Bestimmung von TSH-Autoantikörpern im Serum von Patienten mit Morbus Basedow nach dem erfindungsgemäßen Verfahren konnten gute Werte für den Interassay-Variationskoeffizient von 4 bis 12 % ermittelt werden. Die Intraassay-Präzision liegt deutlich unter 10 %. Das erfindungsgemäße Verfahren eignet sich ausgezeichnet zum automatisierten Nachweis von Autoimmunantikörpern in Patientenproben. Zur Durchführung des erfindungsgemäßen Verfahrens wird jeweils eine Patientenprobe mit einer der drei TSH-Rezeptorchimären umgesetzt und auf den entsprechenden Autoimmunantikörper untersucht.

Beispielhafte Ausführungsformen der erfindungsgemäßen Verfahren werden nachfolgend unter Bezugnahme auf die Abbildungen beschrieben.

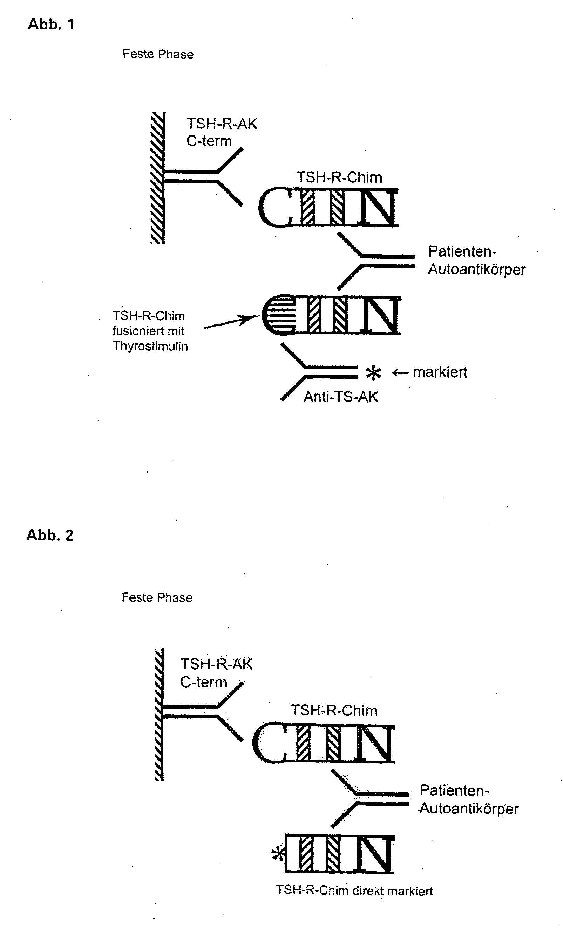

Abb. 1 zeigt eine Ausführungsform des erfindungsgemäßen Verfahrens, in der eine an eine Festphase gebundene trunkierten TSH-Rezeptorchimäre mit einer Patientenprobe in Kontakt gebracht worden ist. Ein Autoimmunantikörper hat an dem entsprechenden Epitop der TSH-Rezeptorchimäre gebunden. Dem Reaktionsgemisch wird dann eine zweite Rezeptorchimäre zugesetzt, die am C-Terminus durch ein hochimmunogenes Peptid modifiziert ist. Das zweite noch freie Epitop im Fab-Teil des Patienten-Autoimmunantikör-pers bindet dann an der zweiten Rezeptorchimäre. Der Nachweis des Autoimmunantikörpers erfolgt mit einem sekundären markierten Antikörper, der am hoch immunogenen Peptid des C-Terminus der zweiten TSH-Rezeptorchimäre bindet.

In der Ausführungsform der Abb. 2 setzt man als zweite trunkierte TSH-Rezeptorchimäre eine solche ein, die an ihrem C-terminalen Ende mit einem Markierungsmittel modifiziert ist. In diesem Fall kann auf einen sekundären markierten Antikörper verzichtet werden.

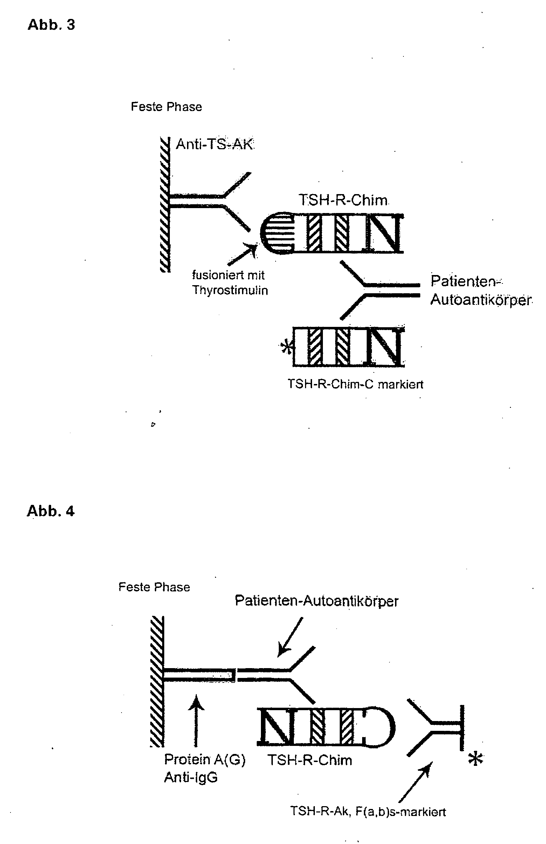

In der Abb. 3 ist eine Verfahrensführung gezeigt, in der der immobilisierende Antikörper ein Antikörper ist, der gegen das Peptid Thyrostimulin gerichtet ist. Die erste TSH-Rezeptorchimäre ist eine trunkierte Rezeptorchimäre, die an ihrem C-terminalen Ende mit dem Peptid Thyrostimulin fusioniert ist. Die zweite TSH-Rezeptorchimäre umfasst ebenfalls nur den extrazellulären Teil der Rezeptorchimäre und ist an ihrem C-terminalen Ende markiert.
Der Autoimmunantikörper bindet sowohl an der ersten als auch an der zweiten TSH-Rezeptorchimäre.

Es ist auch möglich, aus einer Patientenprobe den Nachweis auf das Vorhandensein von mehreren TSH-Autoimmunantikörpern zu führen. Zu diesen Zweck kann beispielsweise zuerst mit Chimäre A oder C der Nachweis blockierender oder neutraler TSH-Autoimmunantikörper durchgeführt werden. In einem weitern Testlauf wird derselben Patientenprobe Chimäre B zugesetzt und nach dem erfindungsgemäßen Verfahren vorgegangen.

Beispielhafte erfindungsgemäße TSH-Rezeptorchimären B und A werden anschließend erläutert.

In Sequenz Nr. 1 bzw. 2 (TSH-Rezeptorchimäre B) stehen die
- Nukleotide 1 bis 51 (Aminosäuren 1-17) für die SEAP-Signalpeptidsequenz,
- Nukleotide 52 bis 1557 (Aminosäuren 18-519) für die SEAP-Sequenz,
- Nukleotide 1558 bis 2280 (Aminosäuren 520-760) für die TSHR-Sequenz der Aminosäuren 21 bis 261 der Chimäre),
- Nukleotide 2281 bis 2298 (Aminosäuren 760-766) für die LHR-Sequenz der Aminosäuren 261 bis 266 der Chimäre),
- Nukleotide 2299 bis 2316 (Aminosäuren 767-772) für 6 Histidine und TAA das Stoppkodon.
   In Sequenz Nr. 3 bzw. 4 (TSH-Rezeptorchimäre B) stehen die
- Nukleotide 1 bis 60 (Aminosäuren 1-20 für die TSHR-Signalpeptidsequenz,
- Nukleotide 61 bis 783 (Aminosäuren 21-261) für die TSHR-Sequenz der Aminosäuren 21 bis 261 der Chimäre,
- Nukleotide 784 bis 846 (Aminosäuren 262-283) für ein hoch immunogenes Epitop.
   In Sequenz Nr. 5 bzw. 6 (TSH-Rezeptorchimäre A) stehen die
- Nukleotide 1 bis 51 (Aminosäuren 1-17) für die SEAP-Signalpeptidsequenz,
- Nukleotide 52 bis 1557 (Aminosäuren 18-519) für die SEAP-Sequenz
- Nukleotide 1561 bis 1998 (Aminosäuren 521-686) für die Aminosäuren 21 bis 166 der LHR-Sequenz der Chimäre,
- Nukleotide 1999 bis 2283 (Aminosäuren 687-781) für die Aminosäuren 166 bis 370 der TSHR-Sequenz (zwischen den Epitopen A und B),
- Nukleotiden 2284 bis 2553 (Aminosäuren 782-891) für die Aminosäuren 261 bis 370 des Epitops der Chimäre, wo blockierende TSH-Autoimmunantikörper binden.
   In der Sequenz 6 bzw. 7 stehen die
- Nukleotide 1 bis 60 (Aminosäuren 1-20) für die TSHR/LHR-Signalpeptidsequenz,
- Nukleotide 61 bis 498 (Aminosäuren 21-166) für die Aminosäuren 21 bis 166 der LHR-Sequenz,
- Nukleotide 499 bis 783 (Aminosäuren 167-261) der Aminosäuren 166 bis 370 des TSH-Rezeptors,
- Nukleotide 784 bis 1113 (Aminosäuren 262-371) für die Aminosäuren 261 bis 370 des Epitops der Chimäre, wo blockierende TSH-Autoimmunantikörper binden,
- Nukleotide 1114 bis 1176 (Aminosäuren 372-392) für ein hochimmunogenes Epitop aus dem cytosolischen Teil des TSH-Rezeptors (codiert durch die Nukleotide 743-763 des TSH-Rezeptors).

In der Sequenz 9 bzw. 10 stehen die
- Nukleotide 1 bis 53 (Aminosäuren 1-18) für die gLUC-Signalpeptidsequenz,
- Nukleotide 54 bis 561 (Aminosäuren 19-187) für die Gausialuciferasesequenz,
- Nukleotide 562 bis 1281 (Aminosäuren 188-427) für die Aminosäuren 21 bis 261 des TS-H-Rezeptors,
- Nukleotide 1282 bis 1302 (Aminosäuren 428-434) für die Aminosäuren 261 bis 266 der LHR-Sequenz in der Rezeptorchimäre und die restlichen Nukleotide für 6 Histidine und das Stoppkodon.

Die Erfindung wird durch die folgenden Beispiele weiter erläutert.

### Beispiele

### Materialien

Das Plasmid pcDNA3-rLHR (B9) wurde von Dr. D.L. Segaloff (The University of Iowa, USA) zur Verfügung gestellt. Das Plasmid pSP-luc-NF wurde von der Promega GmbH (Heidelberg, Deutschland) erworben. Als ECL Western-Blot-Kit und cAMP-RIA-Kit wurden Kits der Amersham GmbH (Braunschweig, Deutschland) verwendet.

Eingesetzt wurden pIRESneo - Expression Vector, pSEAP2-Basic von CLONTECH Laboratories, Inc, Palo Alto, CA USA, Gaussia Luciferase von P.J.K. GmbH, Kleinbittersdorf, Deutschland, Alkaline Phosphatase E. coli von Laboratory voor Monoklonale antistoffen (LMA), Wageningen, Niederlande, Horseradish Peroxidase von Armoracia rusticiana, SYNTHETIC GENE, British Bio-Technology Ltd, UK, h-Thyrostimulin: Nakabayashi et al "Thyrostimulin, a heterodimer of two new human glycoprotein hormone subunits, activates the tyroid-stimulating hormone receptor", J. Clin. 109 (11), 1445-1452.

Die DNA-Primer

| | |
|---|---|
| P1 | (5'-GTCATGCATCAGCTGCTGGTGCTGGCAGTG-3') |
| P2 | (5'-GTCGACGTCGTTATGTGTAAGTTATCACAG-3') |
| P3 | (5'-GTCCTTAAGAAAACACTGCCCTCCAAAGAAAAA-3'9 |
| P4 | (5'-ATCGAGCTCTTCATTCTCCTCAAAGATGGC-3') |
| P5 | (5'-TACGATATCGGAATGGGGTGTTCGTCT-3') |
| P6 | (5'-TATGGATCCTTATTTQGAGGGCAGTGTTTT-3') |
| P7 | (5'-TACGATATCATGCTGCTGCTGCTGCTGCTGCTGGGC-3') |
| P8 | (5'-TACAGCGCTTGTCTGCTCGAAGCGGCC-3') |

wurden von der Firma Interactiva (Ulm, Deutschland) bezogen.

### Zellkultur

HEK293-Zellen wurden in Dulbecco's modifiziertem Eagle's Medium, das mit 10 % fötalem Rinderserum ergänzt war, gezüchtet. Die Zellen wurden in einer 5%igen CO₂-Atmosphäre bei 37 °C kultiviert.

### Konstruktion von TSHR/LH-CGR-Chimären

Drei unterschiedliche Chimären des humanen TSHR, bei dem Teilsequenzen durch entsprechende Sequenzen eines Ratten-LH-CGR ersetzt waren, wurden gemäß der Beschreibung in Biochem Biophys Res Commun. (1991), 179: 70-77 konstruiert. Bei einer der Chimären, die nachfolgend als "Chimäre A" bezeichnet wird, waren die TSHR-Aminosäuren 8 bis 165 durch die vergleichbaren Aminosäuren 10 bis 166 des LH-CGR ersetzt; bei "Chimäre B" waren die TSHR-Aminosäuren 261 bis 370 durch die vergleichbaren Aminosäuren 261 bis 329 eines Ratten LH-CGR ersetzt, während im Falle der "Chimäre, C" sowohl die Aminosäuren 8-165 als auch die Aminosäuren 261 bis 370 durch vergleichbare LH-CGR-Aminosäuren ersetzt waren.

Im Einzelnen wurde dabei von dem Plasmid pcDNA3-rLHR (B9) ausgegangen, das die Sequenz für den Ratten-LH-CGR-Rezeptor enthält. Die DNA-Sequenzen, die für die Aminosäuren 10 bis 165 sowie 261 bis 329 kodierten, wurden nach der PCR-Technik unter Verwendung der Primpaare P1-P2 bzw. P3-P4, die NsiI, AatII,_ BfrI bzw. SacI-Restriktionsstellen enthielten, vervielfältigt, wodurch man NsiI/AatII bzw. BfrI/SacI-PCR-Fragmente erhielt. Parallel dazu wurde ein pTM1-TSHR-FLAG-6HIS-Plasmid, das gemäß DE 196 45 729 oder Exp Clin Endocrinol Diabetes 5: 282-290 (1997) erhalten worden war, mit PstI-AatII oder BfrISacI-Restriktionsendonukleasen verdaut. Die dabei erzeugten Fragmente wurden entfernt und durch die aus dem Ratten-LH-CG-Rezeptor erhaltenen PCR-Fragmenten ersetzt, wodurch die cDNA-Sequenzen für die unterschiedlichen TSHR/LH-CGR-Chimären A, B und C erhalten werden.

Der Vektor pTMl-TSHR-FLAG-&HIS oder der daraus erhaltene neue Vektor mit der TSHR/LH-CGR-DNA (PTM1-TSHR/LH-CGR) wurde mit Ava I linearisiert und die "klebrigen" Enden unter der Verwendung von Klenow-Polymerase aufgefüllt.
Der Vektor pIRESneo wurde mit Cla I linearisiert und die "klebrigen" Enden unter der Verwendung von Klenow-Polymerase aufgefüllt. Das für die Expression bestimmte TSHR bzw. TSHR/LH-CGR Fragment wurde unter Verwendung von Bam HI herausgeschnitten und in die Cla I (aufgefüllte Stelle) / BamH I Stelle des Expressionsvektor pIRESneo subkloniert. So entstehen Expressionsvektoren pIRESneo-TSHR bzw pIRESneo-TSHR/LH-CGR für die Zellentransfektion und Expression der verschiedenen TSH-Rezeptorchimären und auch für Modifikationen der Chimären.

### Herstellung des Extrazellulärteils der TSH-Rezeptorchimären

Die TSH-Rezeptorchimäre B (Wildtyp), die in den Expressionsvektor pIRESneo kloniert ist, wird als Template für die Herstellung und Amplifikation der Nukleotidsequenz mit Polymerase-Kettenreaktion (PCR) für den extrazellulären Teil der TSH-Rezeptorchimäre B benutzt. Die für die PCR erforderlichen beiden Primer enthalten folgende Nukleotidsequenzen: Primer V enthält eine Sequenz von 6 Nukleotiden für das Restriktionsenzym EcoR V und eine Sequenz von 18 Nukleotiden für die N-terminale Peptidsequenz in Chimäre B ohne Signalpeptid. Primer 6 enthält die Nukleotidsequenz für die Aminosäuren 261 bis 266 zusammen mit der Sequenz von 6 Nukleotiden für Bam H1

### Insertion der TSH-Rezeptor-Chimäre B Nukleotidsequenz (nur extrazellulärer Teil) in Expressionsvektor

Das gewonnene PCR-Produkt enthält die Nukleotide, die für die Aminosäuren 21 bis 266 codieren. Diese Sequenz wird über EcoR V und Bam I-Schnittstellen in pIRESneo insertiert.

### Fusion der Nukleotidsequenzen der extrazellulären TSH-Rezeptorchimäre B und des Enzyms SEAP

Zunächst wurden als Voraussetzung für die anschließende Fusion mit pSEAP-2 die beiden folgenden Primer hergestellt. Primer 7 enthält die Nukleotide für die N-terminale Aminosäurensequenz von SEAP und die Nukleotide für EcoR V. Primer 8 enthält die Nukleotide für die c-terminale Aminosäurensequenz von SEAP zusammen mit den Nukleotiden von Ecc 47III. Hiermit erfolgt die Amplifizierung dieses Templats durch eine Polymerase-Kettenreaktion.

### Insertion

Dann erfolgt die Insertion der fusionierten Nukleotidsequenz in pIRESneo-chim B nach dessen Spaltung an der EcoR V-Schnittstelle. Durch Restriktionsanalyse mit dem Enzym EcoR V und Bam HI werden Klone mit der fusionierten Nukleotidsequenz selektiöniert (nach der Wahrscheinlichkeit werden 50 % der Klone SEAP in falscher Orientierung enthalten sein, die dann bei der Restriktionsanalyse sich durch eine kürzere Länge von der korrekten Fusionsnukleotidsequenz unterscheiden.

### Einbau einer sekretorischen Signalpeptidsequenz

Dieser Vorgang erfolgt mit den gleichen gentechnischen Maßnahmen wie oben unter Fusion und Insertion angegeben. Insbesondere wird hier die Signalpeptidsequenz von Transtyrethin verwendet, da es eine hohe Sekretionsleistung für das hochmolekulare Globulin Transthyretin bewirkt.

### Expression und Gewinnung von Fusionsoroteinen TSHR-SEAP bzw. TSHR/LH-CGR-SEAP als. Zellextrakt

Konfluente stabile HEK293-Zellen werden in 10-20 75 cm²-Platten (etwa 20 x 106 Zellen) gezüchtet. Nach Abschaben werden die Zellen in eine phosphatgepufferte Salzlösung (PBS) überführt und viermal mit PBS unter Zentrifugieren bei 2500 U/min gewaschen. Die erhaltenen Zellen wurden in 0,3 ml eines Puffers A (20 mM Hepes-KOH; pH 7,5; 50mM NaCl; 1% Triton X100; 10 % Glycerol) unter Einfrieren und Auftauen lysiert. Die erhaltene Suspension wurde bei 30.000 G 1 Stunde lang zentrifugiert, der Überstand (etwa 8 mg/ml Gesamtprotein) wurde gesammelt und bei -70 °C aufbewahrt.

Der so erhaltene Überstand (Extrakt) kann in Bestimmungsverfahren als TSHR-SEAP bzw. TSHR/LH-CGR-SEAP eingesetzt werden.

### Herstellung von Zellfraktionen

Die HEK193-Zellen werden durch Zentrifugieren bei 1.200 U/min pelletiert. Das erhaltene Zellpellet wurde in 0,3 ml eines Puffers resuspendiert, der in 10 mM Tris-HCl, pH 7,6, 50 mM NaCl, 10 % Glycerol sowie eine Proteaseinhibitorenmischung enthielt. Die Suspension wurde danach bei 4 °C durch 20 Hubbewegungen in einem Glas/Teflon-Homogenisator homogenisiert und dann 15 min bei 800 G und anschließend 1 Stunde lang bei 30.000 G zentrifugiert. Der Überstand (die Cytoplasmafraktion) wurde gesammelt. Das Membranpellet wurde aufgearbeitet, indem es bei 4°C durch 20 Hubbewegungen in eine Glas/Teflon-Homogenisator in 0,3 ml 1% Triton X100 im gleichen Puffer homogenisiert und dann bei 30.000 G 1 Stunde lang zentrifugiert wurde. Der Überstand (Triton X100-Membranextrakt) wurde gesammelt und bei -70°C aufbewahrt.

### Gewinnung der sekretierten extrazellulären Fusions-TSHR-Chimären

Die trunkierten, extrazellulären Domänen der Fusions-TSH-R-Chimären werden von den sie exprimierenden Zellen in den Kulturüberstand sekretiert. Die sekretierten Rezeptor-Proteine werden in Form von bestimmten Verdünnungen im Assaypuffer direkt in den Versuch eingesetzt. Zum Beispiel werden 10 µl extrazelluläre SEAP-TSH-R-Chimäre B aus 5 ml Kulturüberstand in einer Endverdünnung von 1:10 pro Bestimmung benutzt.

### Erstellung einer Standardeichkurve

Zur Erstellung der Standardkurve wurde eine Standardlösung WHO 90/672 mit einer standardisierten Konzentration von Autoimmunantikörper eingesetzt. Die Ausgangslösung des Standards enthält 100 IU/1, die für die Zwecke der Erstellung der Standardkurve verdünnt wurden. Als Nullwert wurde das Serum eine Probanden ohne Autoimmunantikörper in TXBW-Puffer verwendet.

Die eingesetzten TSH-Rezeptor-Chimären B sind fixiert über Antikörper auf Mikrotiterplatten lyophilisiert gelagert und werden vor Gebrauch in Puffer mit Proteinstabilisatoren rekonstituiert. Der Triton X-100-Waschpuffer (TXWP) enthält 0,1% TX-100, 50 mM Tris/HCl pH 8,0, 100 mM NaCl. Der Serumverdünnungspuffer enthält 5% Glucose und 5% Milchpulver in TXWP.

50 µl Probenlösung (Verdünnungen des Standards und Nullprobe) pro Vertiefung werden mit Verdünnungspuffer 1:2 verdünnt und 90 Minuten lang bei Raumtemperatur (etwa 22°C) auf Mikrotiterplatten inkubiert. Es wird viermal mit je 300 µl TXWP gewaschen. Dann erfolgt die Zugabe von 10µl einer 1:100-Verdünnung aus 5 ml Kulturüberstand einer Kulturschale eines Durchmessers von 10 cm von extrazellulärer TSH-Rezeptorchimäre, fusioniert mit einem Enzym SEAP, zu 90 µl TXWP. Anschließend wird unter Schütteln (300-400 UpM) 30 Minuten lang bei zu inkubiert. Dann wird viermal mit 300 µl TXWP gewaschen. Die Bio/Chemolumineszenz wird mit dem Centrol^{®} IA LB 296 Mikrotiterplatten-Meßgerät der Berthold GmbH, Bad Wildbad, Schwarzwald, Deutschland unter Einsatz von Tropix^{®} (Reagenz für ECL (enhanced Chemolumineszenz) von Applied Biosystems, Foster City, CA, USA gemessen.

Die Nachweisgrenze liegt bei etwa 0,2 IU/l. Es besteht eine polynomische Funktion zwischen relativen Lichteinheiten (RLU=Relative Light Unit und den Konzentrationen des Standards für stimulierende Autoantikörper über einen Bereich, der bis mindestens 40 IU/l reicht.

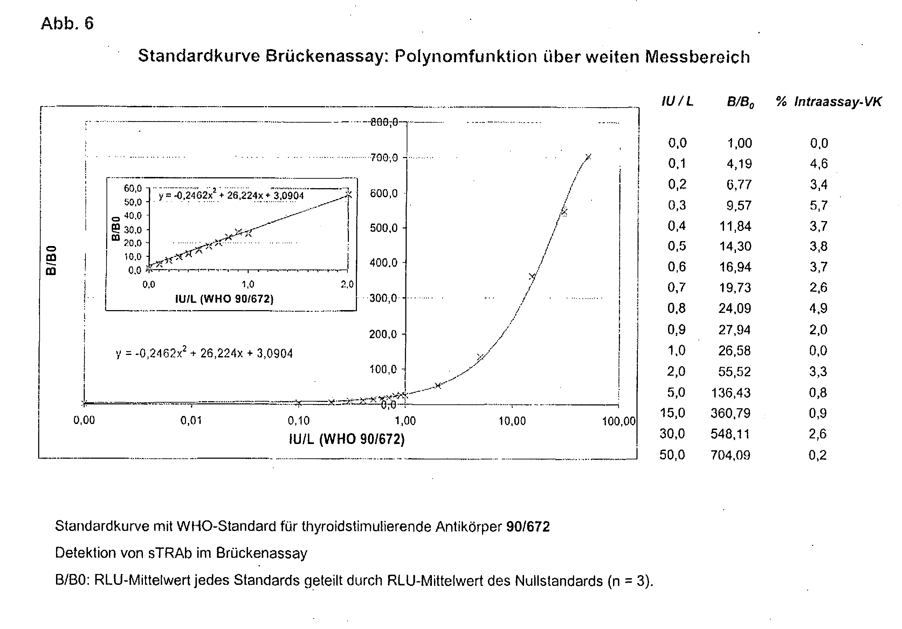

In der Abb. 6 ist auf der Y-Achse das Verhältnis der relativen Lichteinheit (RLU) der in den Proben der Verdünnungsreihe des TSH-Rezeptor-Autoantikörperstandards gemessenen RLU (B=bound) im Verhältnis jeweils zu dem Nullwert eines Probanden ohne Autoimmunantikörper (B0), dargestellt als B/B0. Auf der X-Achse ist die Verdünnung der Proben des Standards angegeben. Die Messwerte, Mittelwerte aus Doppelbestimmungen, sind in der folgenden Tabelle wiedergegeben.

| IU / L | Verdünnungsfaktor NIBSC | RLU Mittelwert | B/B0 | Intraassay-Variationskoeffizient |
|---|---|---|---|---|
| | (100 IU / L) | n = 3 | | |
| 0,0 | -- | 6.882,3 | 1,00 | 0,0 |
| 0,1 | 1000 | 28.847,7 | 4,19 | 4,6 |
| 0,2 | 500 | 46.607,0 | 6,77 | 3,4 |
| 0,3 | 333,3 | 65. 848, 3 | 9, 57 | 5,7 |
| 0,4 | 250 | 81.488,0 | 11,84 | 3,7 |
| 0,5 | 200 | 98.427,3 | 14,30 | 3,8 |
| 0,6 | 166, 7 | 116.567,7 | 16, 94 | 3,7 |
| 0,7 | 142,9 | 135.783,7 | 19,73 | 2,6 |
| 0,8 | 125 | 165.778,3 | 24,09 | 4,9 |
| 0,9 | 111,1 | 192.274,7 | 27,94 | 2,0 |
| 1,0 | 100 | 182.910,3 | 26,58 | 0,0 |
| 2,0 | 50 | 382.131,7 | 55,52 | 3,3 |
| 5,0 | 20 | 938. 959, 7 | 136,43 | 0,8 |
| 15,0 | 6,7 | 2.483.045,7 | 360,79 | 0,9 |
| 30,0 | 3,3 | 3.772.245,0 | 548,11 | 2,6 |
| 50,0 | 2 | 4.845.780,7 | 704,09 | 0,2 |

### Vergleich des erfindungsgemäßen Tests mit dem Kompetitionsassay der "zweiten Generation"

Serum- oder Plasmaproben werden innerhalb von 3 Stunden aus venösem Blut gewonnen. Lagerung bei 4°C über einen zeitraum von 7 Tage oder bei -20°C 1 bis 2 Jahre möglich. Sekundäre Antikörper (siehe oben) werden bei -20°C aufbewahrt und vor dem Einsatz im Test bei Raumtemperatur aufgetaut. TSH-Rezeptorchimären werden auf Mikrotiterplatten lyophilisiert gelagert und vor Gebrauch in Puffer mit Proteinstabilisatoren rekonstituiert. Triton X-100-Waschpuffer (TXWP) enthält 0,1% TX-100, 50 mM Tris/HCl pH 8,0, 100 mM NaCl. Serumverdünnungspuffer enthält 5% Glukose und 5% Milchpulver in TXWP.

50 µl Serum oder Plasma pro Vertiefung werden mit Puffer TXWP 1:2 verdünnt und 9.0 Minuten lang bei Raumtemperatur (etwa 22°C) auf Mikrotiterplatten inkubiert. Dann erfolgt die Zugabe von 10 µl einer 1:10-Verdünnung aus 5 ml Kulturüberstand einer Kulturschale eines Durchmessers von 10 cm von extrazellulärer TSH-Rezeptorchimäre, fusioniert mit einem Enzym SEAP, zu 90 µl TXWP. Anschließend wird unter Schütteln (300-400. UpM) 30 Minuten lang bei 37 °C inkubiert. Dann wird viermal mit 300 µl TXWP gewaschen. Die Bio/Chemolumineszenz wird mit dem Centrol^{®} IA LB 296 Mikrotiterplatten-Meßgerät unter Einsatz von Tropix^{®} gemessen.

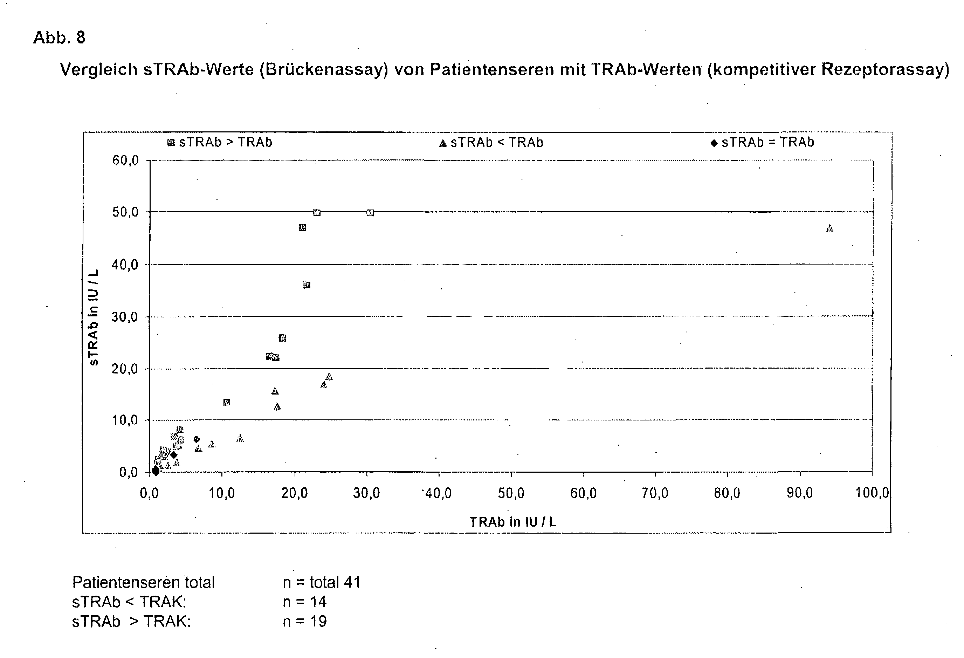

Abb. 8 zeigt vergleichsweise die Messergebnisse, die an Patientenseren mit dem erfindungsgemäßen Verfahren und mit einem Test der so genannten zweiten Generation erzielt wurden. In dem Test der zweiten Generation wurde mit einem vollständigen TSH-Rezeptor - keine Rezeptorchimäre - in einem Kompetitionstest gearbeitet. Dabei wird der TSH-Rezptor an einer Festphase immobilisiert und mit Probeserum, markiertem TSH in Kontakt gebracht. Die Konzentration des eingesetzten TSH wurde variiert. Autoimmunantikörper verdrängen TSH vom Rezeptor. Die durch die Verdrängung bewirkte Signaländerung wird als Messwert herangezogen.

Das Assaydesign des erfindungsgemäßen Tests war das von Abb. 2. Die Durchführung des Kompetionsassays erfolgte entsprechend den Anweisungen des eingesetzten Test-Kits TRAK^{®} der B.R.A.H.M.S AG, 16761 Hennigsdorf, Deutschland.

In der Abb. 8 sind die erfindungsgemäß ermittelten internationalen Einheiten pro Liter auf der Y-Achse und diejenigen nach dem Verfahren der zweiten Generation auf der X-Achse des Diagramms angegeben. Die Auswertung zeigt, dass bei insgesamt 41 Patientenseren die sTRAb-Werte in 14 Fällen niedrieger sind und in 19 Fällen höher als die TRAK-Werte liegen.

Obwohl die Erfindung vorstehend unter Bezugnahme auf bestimmte Ausführungen beschrieben wurde, sind Änderungen und Abwandlungen möglich, die für den Fachmann offensichtlich sind und die den durch die Ansprüche vorgegebenen Schutzumfang nicht verlassen.

## Patentansprüche

1. Verfahren zur differentiellen Bestimmung unterschiedlicher gegen den TSH-Rezeptor (TSH-R) gerichteter Typen von Autoantikörpern in einer Patientenprobe unter Einsatz der TSH-Rezeptorchimären A, B oder C als Bindungsreagens für den Autoantikörper, in denen die für die Bindung von stimulierenden Autoantikörpern (Chimäre A) oder blockierenden Autoantikörpern (Chimäre B), oder beiden (Chimäre C) wesentlichen Sequenzen des Rezeptors durch entsprechende, keine Bindung des jeweiligen Typs von Autoantikörpern bewirkende Sequenzen eines anderen Rezeptors ersetzt sind, **dadurch gekennzeichnet, dass** man
(a) die Patientenprobe in Kontakt bringt mit an eine Festphase gebundenen ersten TSH-Rezeptorchimären A, B oder C, wobei ein Autoimmunantikörper mit einem antigenbindenden Fragment an der ersten TSH-Rezeptorchimäre bindet,
(b) eine trunkierte TSH-Rezeptorchimäre A, B oder C als zweite Rezeptorchimäre hinzufügt, die nur den extrazellulären Teil der TSH-Rezeptorchimäre A, B oder C umfasst, wobei das andere antigenbindende Fragment des Autoimmunantikörpers an der zweiten Rezeptorchimäre bindet und die trunkierte Rezeptorchimäre N-terminal oder C-terminal ein Enzym oder ein anderes Markierungsmittel für den Nachweis aufweist
und
(c) den Nachweis des Autoimmunantiköpers durchführt,

2. Verfahren nach Anspruch 1, **dadurch gekennzeichnet, dass** das Markierungsmittel eine Acridin- oder Rutheniumverbindung oder Fluoresceinisothiocyanat ist.

3. Verfahren nach Anspruch 1, **dadurch gekennzeichnet, dass** das Enzym eine sekretorische alkalische Phosphatase, eine Leuchtkäfer- oder Gausialuciferase oder eine Peroxidase ist.

4. Verfahren nach Anspruch 1, **dadurch gekennzeichnet, dass** das Markierungsmittel eine immunogene Peptidsequenz ist, die durch einen zur Detektion geeigneten sekundären Antikörper erkannt wird.

5. Verfahren nach einem der Ansprüche 1 bis 4, **dadurch gekennzeichnet, dass** die erste TSH-Rezeptorchimäre an ihrem C-terminalen Ende mit einem Peptid fusioniert ist, gegen das ein an die Festphase gebundener immobilisierender Antikörper gerichtet ist.

## Claims

1. A method for differential determination of different types of auto-antibodies directed against the TSH receptor (TSH-R) in a patient sample using the TSH receptor chimeras A, B or C as binding reagent for the auto-antibody, in which the sequences of said receptor being substantial for the binding of stimulating auto-antibodies (chimera A) or blocking auto-antibodies or both (chimera C), are replaced by the corresponding sequences of another receptor, which do not effect binding of the respective type of auto-antibodies,
**characterized in that**
(a) said patient sample is contacted with first TSH receptor chimeras A, B or C bound to a solid phase, wherein an autoimmune antibody binds with an antigen-binding fragment to said first TSH receptor chimera,
(b) a truncated TSH receptor chimera A, B or C is added as a second receptor chimera, which only comprises the extra-cellular part of the TSH receptor chimera A, B or C wherein the other antigen-binding fragment of said autoimmune antibody binds to said second receptor chimera, and the truncated chimera features an enzyme or another labeling agent at its N-terminus or C-terminus for the detection reaction, and
(c) the detection of the autoimmune antibody is carried out.

2. The method according to claim 1, **characterized in that** said other labeling agent is an acridine or ruthenium compound or fluorescin isothiocyanate.

3. The method according to claim 1, **characterized in that** said enzyme is a secretory alkaline phosphatase, a glow-worm or Gaussia luciferase, or a peroxidase.

4. The method according to claim 1, **characterized in that** said labeling agent is an immunogenic peptide sequence that is recognized by a secondary antibody suitable for detection.

5. The method according to one of the claims 1 to 4, **characterized in that** said first TSH receptor chimera is fused with its C-terminal end to a peptide, against which a solid phase bound, immobilizing antibody is directed.

## Revendications

1. Procédé pour l'identification de différents types d'anticorps contre le récepteur de la TSH (TSH-R) dans un échantillon, prélevé sur un patient, en utilisant les récepteurs chimères de la TSH A, B ou C en tant que réactif de liaison pour l'autoanticorps, dans lesquels, les séquences du récepteur, essentielles pour la liaison d'autoanticorps stimulants (chimère A) ou d'autoanticorps bloquants (chimère B) ou des deux (chimère C), sont remplacées par des séquences correspondantes d'un autre récepteur, qui ne provoquent aucune liaison du type d'autoanticorps respectif, **caractérisé en ce que**
a) un échantillon, prélevé sur le patient, est mis en contact avec un premier récepteur chimère de la TSH lié à une phase fixe, un autoanticorps avec un fragment de liaison à un antigène, se liant au premier récepteur chimère de la TSH,
b) un récepteur chimère tronqué A, B ou C, qui ne comprend que la partie extracellulaire du récepteur chimère de la TSH A, B ou C, est ajouté en tant que deuxième récepteur chimère, l'autre fragment de l'autoanticorps se liant au deuxième récepteur chimère, et le récepteur chimère tronqué N-terminal ou C-terminal présentant un enzyme ou un autre moyen de marquage pour la détection.
c) la détection de l'autoanticorps est exécutée.

2. Procédé selon la revendication 1, **caractérisé en ce que** le moyen de marquage est une combinaison d'acridine ou de ruthénium ou un thiocyanate de fluorescéine.

3. Procédé selon la revendication 1 **caractérisé en ce que** l'enzyme est une phosphatase alcaline sécrétoire, une luciférase de luciole ou gaussia ou une peroxydase.

4. Procédé selon la revendication 1 **caractérisé en ce que** le moyen de marquage est une séquence de peptides immunogènes, qui est détectée par un anticorps secondaire, apte à la détection.

5. Procédé selon l'une des revendications 1 à 4,
**caractérisé en ce que** le premier récepteur chimère de la TSH est fusionné, à son extrémité terminale C, avec un peptide, contre lequel est dirigé un anticorps immobilisant, lié à la phase fixe.
